# EUROPEAN PATENT APPLICATION

(11) **EP 1 142 545 A2**
(43) Date of publication of application: **10.10.2001**
(21) Application number: 01500097.9
(22) Date of filing: 06.04.2001
(51) Int. Cl.: A61F 13/02

(54) **Sanitary adhesive strip**

(30) Priority: 07.04.2000 ES 200000946 U
(71) Applicant: Catarineu Guillén, D. Leonardo, 08190 La Floresta (Barcelona) (ES)
(72) Inventor: Catarineu Guillén, D. Leonardo, 08190 La Floresta (Barcelona) (ES)
(74) Representative: Duran Moya, Carlos

(57) **Abstract**

The sanitary adhesive strip comprises a support which is provided, on one of its faces, with a luminescent material which is capable of projecting light in the dark after it has been exposed to the light for a few seconds and, on the other face, with a sanitary material and adhesive regions for securing the strip.

## Description

The present invention relates to a sanitary adhesive strip which has substantial advantages over the currently known adhesive strips for sanitary use.

The adhesive strip for sanitary use to which the present invention relates comprises an impermeable plastics strip which has an antiseptic or therapeutic covering on one face and, on the other face, which will constitute the upper face, a covering of luminescent material which is capable of shining in the dark after being illuminated for a short time. In practice, this involves a significant improvement and even a substantial change in this type of sanitary adhesive strip, since, by shining in the dark, it enables a novel effect in the form of an indicator to be obtained, permitting the detection of the location of the lesion or wound covered by the strip, even in the dark. This prevents undesired friction between the user and either objects or another person who, because of the adhesive strip to which the present invention relates, will notice that the other person has a wound in a specific region thanks to the nocturnal visibility of the adhesive.

The strip to which the present invention relates also has the aspect of a children's game and therefore it will be more accepted when its users are young children since it will give them an appearance different from that normally provided by sanitary strips. Children will therefore appreciate them for their attractive and original outer appearance, completely changing the normal degree of acceptance which small users display in respect of adhesive strips having purely curative purposes.

In order further to increase the attractive nature of the adhesive strips to which the present invention relates, they may have, on the face provided with a luminescent (phosphorescent) covering, drawings or inscriptions which will be seen as negatives in the dark and which correspond to persons or objects usual in children's stories, including, for example, spaceships, wizards' faces, animals or the like, or advertising images, and therefore they can even be used as simple adhesives on the head or fingertips, using the luminescent effect for games, night-time puppet theatre using the hands, etc.

Some explanatory drawings of the adhesive strip for sanitary use to which the present invention relates are appended by way of non-limiting explanatory example for a better understanding of the invention.

Figure 1 is a perspective view of an adhesive strip for curative use according to the present invention, showing the luminescent face.

Figure 2 is a perspective view of the same strip from the side which has the regions with a curative and adhesive coating.

Figure 3 is a view in longitudinal section.

Figure 4 shows the application of the adhesive strip to a user's finger.

As will be appreciated from the drawings, the adhesive strip to which the present invention relates has a main support strip 1. The upper face of said main support strip carries the covering layer 2 of a luminescent (phosphorescent) type, which is optionally provided with graphic or letter-form motifs 3 in order to increase its visual impact, and the opposite face of the support 1 has a layer 4, which may or may not carry an antiseptic or curative sanitary material 9, and end regions indicated by the numerals 5 and 6 which correspond to the regions provided with adhesive which enable the strip to be secured to the human body in regions in the immediate vicinity of the wound which it is desired to protect, or on other parts of the human body if a purely decorative effect is sought.

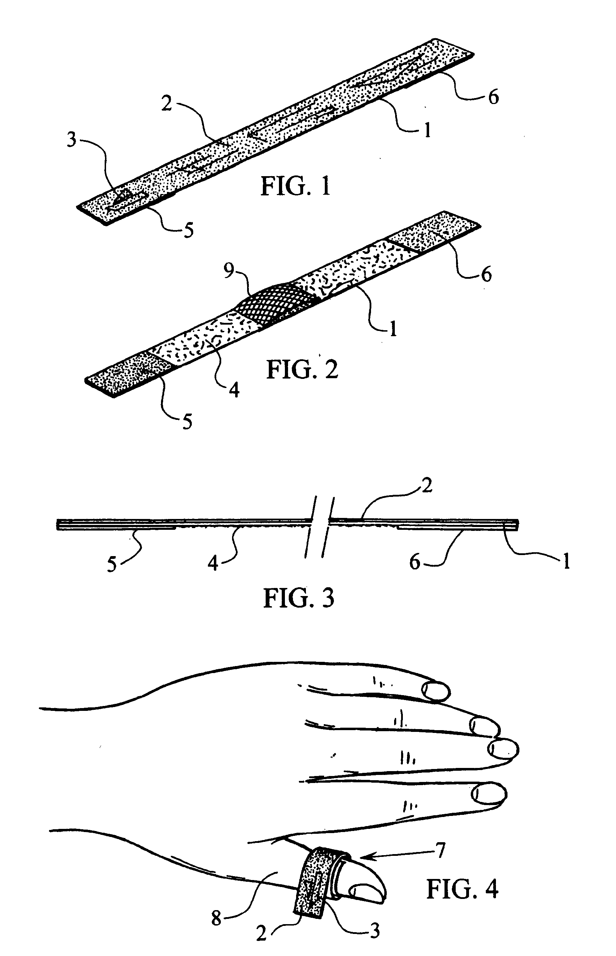

The application of the strip, which is generally indicated 7 in Figure 4, to the user's hand 8 enables the luminescent layer 2 to be arranged on the outside, the graphic motifs 3 being clearly visible.

By producing the adhesive strip in the form which has been explained, the effect of protecting and treating small wounds, which can be achieved by the curative covering of the lower face 4, is combined with the versatile luminescent decorative effect constituted by the layer 2 of the upper face, with the optional graphic motifs 3.

As will be appreciated, although the form of the present invention has been demonstrated by way of example, as shown in the drawings, it could be substantially varied without departing from the scope of the invention. Thus, for example, the luminescent layer 2 could extend over only a portion of the upper face of the curative strip or over the entire face, as indicated in the drawings. Equally, it would be possible to manufacture the support of the strip separately from the covering, the latter being applied later to one of the faces of the strip, or it would be possible to produce the support in such a manner that it carries incorporated in its upper face the luminescent material extending over the entire upper face or over only a portion thereof. Other examples of variations in the size and arrangement of the curative, adhesive and luminescent layers could also come within the scope of the present invention.

## Claims

1. Sanitary adhesive strip, **characterised in that** it comprises a main support strip which is provided, on one of its faces, with a luminescent material capable of projecting light in the dark after it has been exposed to the light for a few seconds and, on the other face, with a sanitary material and adhesive regions for securing the sanitary strip.

2. Sanitary adhesive strip according to claim 1, **characterised in that** the luminescent material extends over the entire upper face of the strip.

3. Sanitary adhesive strip according to claim 1, **characterised in that** the luminescent material extends over only a portion of the upper face of the strip.

4. Sanitary adhesive strip according to the preceding claims, **characterised in that** the luminescent material carries graphic motifs which are visible separately.

5. Sanitary adhesive strip according to claim 1, **characterised in that** the luminescent material is incorporated in the support of the strip in the form of a covering.

6. Sanitary adhesive strip according to claim 1, **characterised in that** the luminescent material is embedded partially or totally in the upper face of the support of the strip.

7. Sanitary adhesive strip according to claim 1, **characterised in that**, on the face opposite the luminescent face, it may or may not carry a sanitary material, the strip being intended to secure other sanitary materials, such as cotton wool, bandages, gauzes and the like.
